# FASCICULE DE BREVET EUROPEEN

(11) **EP 2 173 190 B1**
(45) Date de publication et mention de la délivrance du brevet: **13.04.2016**
(21) Numéro de dépôt: 08827683.7
(22) Date de dépôt: 09.07.2008
(51) Int. Cl.: A23P 10/30, A23L 29/212, A23L 29/30, C08L 3/02

(54) **UTILISATION DE MALTODEXTRINE DE POIS ET/OU DE SIROP DE GLUCOSE DE POIS POUR L'ENCAPSULATION DE COMPOSES HYDROPHOBES**
VERWENDUNG VON ERBSENMALTODEXTRIN UND/ODER ERBSENGLUCOSESIRUP ZUR EINKAPSELUNG WASSERABWEISENDER VERBINDUNGEN
USE OF PEA MALTODEXTRIN AND/OR OF PEA GLUCOSE SYRUP FOR ENCAPSULATING HYDROPHOBIC COMPOUNDS

(30) Priorité: 19.07.2007 FR 0705232
(43) Date de publication de la demande: 14.04.2010
(73) Titulaire: Roquette Frères, 62136 Lestrem (FR)
(72) Inventeur: BOURSIER, Bernard, F-62138 Violaines (FR)
(74) Mandataire: Cabinet Plasseraud
(86) Numéro de dépôt international: PCT/FR2008/051284
(87) Numéro de publication internationale: WO 2009/024690

(56) Documents cités:
- EP-A- 0 820 702
- EP-A- 0 953 578
- EP-A- 1 304 044
- WO-A-2004/064540
- FR-A- 2 865 111
- US-B1- 6 187 351
- REINECCIUS G A: "THE SPRAY DRYING OF FOOD FLAVORS" DRYING TECHNOLOGY, TAYLOR & FRANCIS, PHILADELPHIA, PA, US, vol. 22, no. 6, juin 2004 (2004-06), pages 1289-1324, XP001200326 ISSN: 0737-3937 cité dans la demande
- INGLETT G E ET AL: "ENCAPSULATION OF ORANGE OIL" ACS SYMPOSIUM SERIES, WASHINGTON, DC, US, vol. 370, 1988, pages 29-36, XP000870453 ISSN: 0097-6156 cité dans la demande
- HOOVER R ET AL: "composition, structure, functionality and chemical modification of legume starches: a review" CANADIAN JOURNAL OF PHYSIOLOGY AND PHARMACOLOGY, OTTAWA, ONT, CA, vol. 69, 1991, pages 79-92, XP008089410 cité dans la demande

## Description

La présente invention concerne l'utilisation de maltodextrines et/ou de sirops de glucose issus d'amidons de légumineuses pour l'encapsulation de composés organiques en vue de l'inclusion, de la stabilisation et de la protection de composés organiques labiles et/ou volatils, et notamment de substances hydrophobes, en particulier de substances aromatiques.

Il est connu de l'homme du métier des industries alimentaires que les composés volatils présents dans les produits contribuent à leur sapidité. Les arômes sont généralement des mélanges de substances aromatiques comprenant principalement des molécules volatiles. La volatilité et/ou la labilité de certaines molécules présentes dans les produits alimentaires peuvent être responsables d'une variation des saveurs et du goût des aliments au cours du temps. De ce fait, les industries alimentaires font souvent le choix d'une augmentation de la concentration de ces arômes afin de pallier leur dégradation ou disparition au cours du temps. Certains de ces composés étant onéreux, l'utilisation de grandes quantités d'arômes pose toutefois un problème de coût.

Par « arôme » on « substance aromatique » on entend un composé conférant une odeur ou un goût à la composition à laquelle il est ajouté. On peut citer à titre d'exemple les oléorésines d'épice, les huiles essentielles des saveurs alliacées ; les extraits botaniques ; les extraits botaniques de saveur ; les hydrolysats de protéines.

L'arôme peut être sous forme d'huile, de solution non aqueuse ou d'émulsion.

Le terme « labile » décrit des composés instables dont l'interaction avec l'environnement entraîne la dégradation, la perte de fonction ou la destruction. Ainsi, certaines molécules peuvent présenter une sensibilité aux agents extérieurs tels que la chaleur, la lumière, l'oxygène de l'air ou l'humidité. Cette sensibilité peut être responsable d'une dégradation ou d'une conversion de la molécule en un composé indésirable dès l'étape de formulation ou lors de la production ou du stockage des produits alimentaires, les rendant ainsi impropres à la consommation.

Par conséquent, ces composés labiles doivent être conditionnés de manière appropriée afin de garantir à la fois
- une bonne conservation sans altération de leurs propriétés organoleptiques et
- leur disponibilité pour l'aromatisation de l'aliment dans lequel ils sont présents.

De nombreux procédés d'encapsulation, de micro-encapsulation ou de piégeage ont été développés afin de protéger les molécules volatiles et/ou labiles d'un arôme pendant sa fabrication, pendant le procédé de fabrication du produit alimentaire ou au cours du stockage et de l'utilisation de celui-ci.

Une problématique similaire se rencontre dans le domaine pharmaceutique où l'encapsulation est utilisée fréquemment pour résoudre les problèmes de labilité, de solubilisation de composés hydrophobes, de biodisponibilité ou d'amertume de certains principes actifs. L'encapsulation permet en outre une libération lente et contrôlée des principes actifs. Bien entendu, les agents d'encapsulation doivent être biocompatibles et biorésorbables.

Le principe actif à encapsuler peut être incorporé tel quel dans l'agent d'encapsulation, c'est-à-dire sous sa forme native, solide ou liquide. On peut également envisager de l'incorporer sous la forme d'une émulsion ou suspension aqueuse. Cette émulsion aqueuse peut être obtenue par émulsification du principe actif pur ou du principe actif préalablement dissous dans une huile appropriée de type huile de silicone par exemple.

Les agents d'encapsulation les plus utilisés sont généralement de nature osidique : l'amidon de blé, de pomme de terre, de maïs et leurs dérivés (amidons modifiés, dextrines, maltodextrines, sirops de glucose, dextrose, polyols...), la gomme arabique qui est le support d'encapsulation le plus utilisé, le saccharose, les cyclodextrines, la cellulose et ses dérivés, les gommes de type alginate, l'agar-agar ou les carraghénanes.

Dans tous les procédés de protection par encapsulation, l'agent d'encapsulation doit présenter les caractéristiques suivantes :
- ne pas modifier les caractéristiques des produits encapsulés,
- être sans odeur,
- avoir une faible viscosité, même à forte concentration,
- stabiliser l'émulsion en cours de séchage,
- être non toxique et comestible,
- avoir une faible hygroscopicité,
- avoir une capacité à libérer progressivement le matériau actif encapsulé et
- avoir un faible coût.

Il existe diverses techniques d'encapsulation choisies en fonction de la destination du produit à encapsuler ou de son utilisation.

Un premier système, appelé système matriciel ou d'enrobage comprend l'inclusion d'une substance dans une matrice solide par occlusion ou adsorption. Cette encapsulation s'effectue par exemple par séchage par atomisation ou séchage par dispersion, au cours duquel les arômes sont émulsionnés dans une phase continue adaptée, puis séchés par pulvérisation dans un courant d'air chaud. Parmi les agents d'encapsulation les plus utilisés pour cette technique, on trouve la gomme arabique, les maltodextrines de maïs, de blé, de tapioca ou de pomme de terre, le dextrose, le lactose et la gélatine. On décrit aussi d'autres technologies telles que la pulvérisation à froid, la granulation, l'extrusion ou l'enrobage en lit fluidisé.

Un second système appelé encapsulation membranaire consiste à entourer les composés à encapsuler d'un film continu de polymères, de lipides, de glucides ou de polysaccharides. Les techniques utilisées sont par exemple la coacervation, la co-extrusion ou l'utilisation de liposomes.

La technique d'extrusion est décrite dans le brevet US 6.187.351 qui décrit l'utilisation de différents polymères, parmi lesquels figurent les amidons et les maltodextrines de maïs, de riz, de blé ou de tapioca.

Le document EP 1.304.044 décrit l'utilisation de sucres, d'amidons modifiés, de maltodextrines et d'autres polymères en association avec un dérivé de cellulose.

Les maltodextrines et les sirops de glucose sont classiquement obtenus par hydrolyse acide et/ou enzymatique d'amidon. Ils peuvent être utilisés comme agent d'encapsulation et renferment un mélange complexe de saccharides linéaires et ramifiés. Du point de vue réglementaire, les maltodextrines ont un équivalent dextrose (DE) de 1 à 20. Les sirops de glucose ont un DE supérieur à 20.

La qualité d'une encapsulation peut être évaluée par exemple en mesurant la protection du composé encapsulé par l'agent d'encapsulation contre l'oxydation.

On a ainsi constaté que, lors de l'encapsulation de limonène par des maltodextrines de maïs ou des sirops de glucose de maïs de DE variés, l'oxydation de l'arôme au cours du temps diminuait lorsque le DE des de l'agent d'encapsulation augmentait (Reineccius G. A. (1988) « Spray drying of Food Flavors" In "Flavor Encapsulation," eds. Sara J. Risch and Gary A. Reineccius, chap. 7, pp. 55-66.). Autrement dit, la protection d'un arôme par encapsulation est meilleure avec des sirops de glucoses de maïs (de DE important) qu'avec des maltodextrines de maïs (de faible DE). De plus, l'encapsulation est meilleure avec les maltodextrines de maïs les plus fortement hydrolysées.

Par ailleurs, une étude de la qualité d'encapsulation par des maltodextrines d'origines végétales diverses a montré que, lorsque les DE de dérivés d'amidon (maltodextrines, sirops de glucose) de maïs, de riz, de tapioca ou de pomme de terre augmentent, la protection des composés encapsulés vis-à-vis de l'oxydation augmente, quelque soit l'origine végétale de l'amidon. En outre, les maitodextrines de maïs, de tapioca ou de riz de haut DE, voire les sirops de glucoses de blé, de tapioca et de riz sont des agents d'encapsulation permettant une meilleure protection face à l'oxydation (Inglett G. E., Gelbman P., and Gary A. Reineccius (1988) "Encapsulation of Orange Oil: Use of Oligosaccharides from α-Amylase Modified Starches of Maize, Rice, Cassava, and Potato." In "Flavor Encapsulation," eds. Sara J. Risch and Gary A. reineccius, chap. 4, pp. 29-36). Ces observations ont été admises comme principe général.

L'art antérieur décrit aussi l'utilisation de cyclodextrines, de dextrines ou d'amylose en tant qu'agent d'encapsulation.

Ces molécules forment des complexes d'inclusion avec les molécules à encapsuler qui sont piégées soit entre des molécules de l'agent encapsulant formant une organisation cristalline, soit dans une cavité formée par une structure de la molécule d'agent d'encapsulation. Les techniques principalement utilisées avec ces agents d'encapsulation sont le pétrissage, la cristallisation et la lyophilisation.

Dans le domaine alimentaire, ces techniques permettent avantageusement d'obtenir des poudres hydrophiles solubles dans l'eau, contenant des composés hydrophobes ou une augmentation de la stabilité thermique des arômes au cours de la cuisson. Elles permettent, par leur caractère réversible, une bonne complexation en milieu concentré et la libération des molécules encapsulées en milieu aqueux dilué ou après contact avec la salive.

La β-cyclodextrine est la plus utilisée des cyclodextrines et la plus économique à produire. Cependant, l'utilisation des cyclodextrines est très réglementée.

Afin de s'affranchir de ces contraintes réglementaires, il a été envisagé de développer l'utilisation d'amylose en tant qu'agent d'encapsulation formant des complexes d'inclusion. L'amylose s'organise en hélices dont la face extérieure est hydrophile en raison de la présence des groupes hydroxyle et la face intérieure est hydrophobe en raison de la présence des atomes d'hydrogène. Cette structure en hélice confère à l'amylose les caractéristiques nécessaires à l'encapsulation de principes actifs hydrophobes ou d'arômes.

L'utilisation d'amylose pur n'est toutefois pas envisageable au stade industriel du fait de leur grande propension à la cristallisation ou à la rétrogradation.

L'utilisation d'amidons riches en amylose (amidons contenant plus de 50% d'amylose) pour l'encapsulation comporte aussi diverses contraintes puisqu'ils nécessitent des conditions de préparation et d'utilisation très strictes. En effet, ces amidons rétrogradent rapidement en raison de leur richesse en amylose. De plus, ils nécessitent des températures de cuisson très élevées, de l'ordre de 120°C. Afin d'éviter les phénomènes de rétrogradation, l'encapsulation doit s'effectuer à des températures élevées, de l'ordre de 90 à 100°C. Or, à ces températures, les composés labiles et/ou volatils sont dégradés ou s'évaporent. Pour réduire leur température de rétrogradation, les amidons contenant plus de 50% d'amylose sont généralement modifiés par fixation de groupements chimiques. Ces amidons sont dits « stabilisés ».

Par « stabilisation » de l'amidon, on entend toutes les opérations connues de l'homme du métier visant à ralentir ou freiner la rétrogradation de l'amidon. La stabilisation est obtenue par substitution des fonctions hydroxyle de l'amidon, par estérification ou éthérification. Elle peut également être obtenue par oxydation. Ces traitements de stabilisation sont notamment l'hydroxypropylation, l'acétylation, la phosphatation et l'oxydation.

La stabilisation d'amidons riches en amylose permet d'abaisser leur température de rétrogradation à 50 - 60°C mais réduit en revanche leur aptitude à former des complexes d'inclusion.

Dans le contexte particulier de l'encapsulation d'arômes de liquides alcoolisés tels que le vin, le document EP 820 702 décrit l'utilisation d'un amidon de pois comme agent d'encapsulation par atomisation ou par lyophilisation. L'amidon de pois est décrit comme conférant à l'encapsulation des qualités de rétention des arômes sans rétention d'alcool. La présence de longues chaînes polysaccharidiques est la caractéristique essentielle pour que l'amidon de pois puisse encapsuler les arômes selon l'invention décrite. En effet, ce document EP 820 702 exclut l'utilisation d'un amidon de pois hydrolyse et tout particulièrement l'utilisation de maltodextrines et de sirops de glucose de pois comme agent d'encapsulation. D'après ce document, ces formes hydrolysées de l'amidon comporteraient des chaînes de sucres trop courtes pour permettre une encapsulation.

Le but de la présente invention est d'élargir le spectre des agents d'encapsulation et des techniques d'encapsulation utilisables avec un même agent d'encapsulation. L'agent d'encapsulation doit être facile à utiliser, non toxique et permettre une libération contrôlée du composé encapsulé.

La présente invention est basée sur la découverte, allant contre les conclusions du EP 820 702, que certains produits d'hydrolyse d'amidons de légumineuses, et notamment de l'amidon de pois, étaient d'excellents d'agents d'encapsulation de substances hydrophobes, notamment de substances aromatiques.

La Demanderesse, au cours de ses essais visant à proposer de nouveaux agents d'encapsulation, a constaté avec surprise que les maltodextrines et sirops de glucose dérivés d'amidons de légumineuses avaient un comportement atypique, différent de celui des produits équivalents dérivés d'amidons de maïs, de riz, de blé ou de tapioca, qui se caractérisait par le fait que leur pouvoir de protection des composés encapsulés vis-à-vis de l'oxydation était d'autant meilleur que leur DE était faible.

La Demanderesse a en outre constaté que les maltodextrines et/ou sirops de glucose dérivés de légumineuses protégeaient mieux certains composés encapsulés contre la dégradation oxydative que ne le faisaient les produits correspondants dérivés d'autres amidons.

La présente invention a par conséquent pour objet l'utilisation d'une maltodextrine et/ou d'un sirop de glucose obtenus, par hydrolyse acide ou enzymatique, à partir d'un amidon de pois présentant une teneur en amylose comprise entre 25 % et 50%, exprimé en poids sec par rapport au poids sec d'amidon, pour l'encapsulation de composés organiques hydrophobes.

L'amidon de pois à partir duquel sont préparés la maltodextrine et le sirop de glucose, présente de préférence une teneur en amylose comprise entre 30 % et 40%, en particulier comprise entre 35 % et 40%, et plus préférentiellement entre 35 % et 38%, ces pourcentages étant exprimés en poids sec par rapport au poids sec d'amidon.

Par «légumineuse» on entend au sens de la présente invention toute plante appartenant aux familles des césalpiniacées, des mimosacées ou des papilionacées et notamment toute plante appartenant à la famille des papilionacées comme, par exemple, le pois, le haricot, la fève, la féverole, la lentille, la luzerne, le trèfle ou le lupin.

Cette définition inclut notamment toutes les plantes décrites dans l'un quelconque des tableaux contenus dans l'article de R. HOOVER *et al*., 1991 (HOOVER R. (1991) « Composition, structure, functionality and chemical modification of legume starches : a review » Can. J. Physiol. Pharmacol.,69 pp. 79-92).

Le pouvoir encapsulant est classiquement déterminé par l'étude de l'évolution de l'oxydation du composé encapsulé en fonction du temps et dans des conditions maîtrisées (Reineccius G. A. (1988) « Spray drying of Food Flavors" In "Flavor Encapsulation," eds. Sara J. Risch and Gary A. Reineccius, chap. 7, pp. 55-66.). Cette mesure permet d'apprécier la qualité de la barrière formée par l'agent d'encapsulation autour du produit encapsulé.

Comme indiqué précédemment, l'augmentation du pouvoir encapsulant et du pouvoir protecteur (antioxydant) des maltodextrines et des sirops de glucose utilisés selon l'invention, lorsque leur DE diminue, n'est pas observée classiquement dans le cas d'une encapsulation par des maltodextrines et/ou sirops de glucose d'autres origines végétales. Pour le maïs, le riz , le tapioca ou la pomme de terre, le phénomène inverse est observé (Reineccius G. A. (1988) « Spray drying of Food Flavors" In "Flavor Encapsulation," eds. Sara J. Risch and Gary A. Reineccius, chap. 7, pp. 55-66.).

On peut citer à titre d'exemples de techniques d'encapsulation de composés par les maltodextrines et les sirops de glucose utilisés dans la présente invention, l'atomisation, la granulation, l'enrobage, le pétrissage, l'extrusion, la lyophilisation, la cristallisation et tout type de techniques d'encapsulation traditionnelles.

L'agent d'encapsulation contenant le produit encapsulé est destiné à l'industrie pharmaceutique, à l'industrie cosmétique, à l'industrie alimentaire, à l'industrie des papiers et non-tissés, le textile, les surodorants et désodorisants, la détergence ou le phytosanitaire. En effet, l'agent d'encapsulation selon l'invention permet une encapsulation de composés variés de caractéristiques physicochimiques et de tailles différentes, tels que notamment des vitamines, des édulcorants intenses, colorants, matières grasses, des actifs lipophiles, hydrophiles, des molécules hydrophiles ou hydrophobes voire des protéines.

Les maltodextrines et sirops de glucose déshydratés utilisés selon l'invention présentent l'avantage d'être faciles à obtenir car, du fait de la faible teneur en amylose de l'amidon dont ils sont issus, les phénomènes de rétrogradation sont observés à des températures plus basses que pour des amidons riches en amylose, c'est-à-dire contenant plus de 50% en amylose.

La rétrogradation des amidons de pois commence seulement à 60-70°C alors qu'à même concentration, les amidons riches en amylose rétrogradent dès 90-100°C. Cette basse température de rétrogradation autorise l'utilisation d'une hydrolyse enzymatique qui n'est pas envisageable pour certains amidons de maïs notamment.

En effet, afin d'éviter la rétrogradation de ces amidons, le manipulateur se place à une température très élevée notamment une température de l'ordre de 100-110°C à laquelle les enzymes classiquement utilisées sont dénaturées par la chaleur et donc inactivées.

Ainsi, les maltodextrines et sirops de glucose utilisés selon la présente invention sont préparés par hydrolyse acide ou hydrolyse enzymatique ménagée d'amidon de pois. Pour les préparer, l'homme du métier se référera aux techniques connues de l'état de la technique.

Dans certains procédés d'encapsulation de la présente invention, les sirops de glucose sont utilisés sous forme déshydratée. Cette déshydratation se fait selon des techniques familières à l'homme du métier.

Selon une variante avantageuse, le pois est une variété de pois donnant des graines contenant au moins 25 %, de préférence au moins 40 %, en poids d'amidon (sec/sec).

Cette richesse en amidon confère à la variété de pois selon l'invention, un intérêt majeur concernant notamment le rendement d'obtention de l'agent d'encapsulation. En conséquence, les maltodextrines et/ou les sirops de glucose selon l'invention sont à la fois de fabrication aisée et peuvent être obtenus avec un haut rendement.

Le terme « pois » étant ici considéré dans son acception la plus large et incluant en particulier :
- toutes les variétés sauvages de « pois lisse » (« smooth pea »), et
- toutes les variétés mutantes de « pois lisse » et ce, quelles que soient les utilisations auxquelles on destine généralement lesdites variétés (alimentation humaine, nutrition animale et/ou autres utilisations).

Lesdites variétés mutantes sont notamment celles dénommées « mutants rb », « mutants rug 3 », « mutants rug 4 », « mutants rug 5 » et « mutants lam » tels que décrits dans l'article de C-L HEYDLEY et al. (HEYDLEY C-L (1996) « Developing novel pea starches » Proceedings of the Symposium of the Industrial Biochemistry and Biotechnology Group of the Biochemical Society, pp. 77-87).

Les sirops de glucose utilisés dans la présente invention présentent de préférence un dextrose équivalent inférieur à 30.

Les maltodextrines de pois utilisées dans la présente invention présentent avantageusement un dextrose équivalent (DE) inférieur à 18 et de préférence compris entre 3 et 7. Ce sont en effet ces produits de faibles DE qui se sont avérés être d'excellents agents d'encapsulation fournissant une protection optimale des composés encapsulés contre l'oxydation.

Les maltodextrines utilisées dans la présente invention sont de préférence totalement solubles dans l'eau sans formation de gel aqueux.

Les composés à encapsuler selon la présente invention sont choisis de préférence parmi les arômes, les colorants, les vitamines liposolubles, les molécules odorantes, les édulcorants intenses, les matières grasses.

On peut citer en particulier les acides gras, les monoglycérides, le décanal, l'octanal, l'hexanal, le butanol, la menthone, la fenchone, le limonène, le naphtol, les oléorésines d'épice, les huiles essentielles des saveurs alliacées, l'aldéhyde benzoïque, le diacétyle (2,3-butanedione), la vanilline, le thymol, le menthol, le camphre, le géraniol, la carvone, la δ-heptalactone, la δ-nonalactone, la δ-decalactone, la δ-dodecalactone, la γ-decalactone, la γ-dodecalactone et la quinoléine.

La présente invention a également pour objet un procédé d'encapsulation comprenant
- la dissolution d'une maltodextrine et/ou d'un sirop de glucose obtenus, par hydrolyse acide ou enzymatique, à partir d'un amidon de pois présentant une teneur en amylose comprise entre 25 % et 50%, exprimé en poids sec par rapport au poids sec d'amidon, dans un solvant aqueux contenant un agent émulsionnant,
- l'addition d'une solution du composé organique hydrophobe à encapsuler dans un solvant organique,
- l'émulsification du mélange obtenu par application de forces de cisaillement, et
- le séchage de l'émulsion obtenue.

Le séchage de l'émulsion peut se faire selon n'importe quelle technique appropriée. On peut citer par exemple l'atomisation et la pulvérisation en lit fluidisé.

Dans un autre procédé selon l'invention, on utilise l'agent d'encapsulation non pas à l'état dissous dans l'eau, mais à l'état solide (poudre). Autrement dit, l'agent d'encapsulation est mis en présence d'une faible quantité d'eau, insuffisante pour le dissoudre complètement mais suffisante pour permettre l'obtention d'une pâte. Cette pâte est ensuite mélangée, par pétrissage et/ou malaxage, avec le composé à encapsuler, à l'état de poudre ou à l'état dissous dans un solvant approprié.

Un tel procédé d'encapsulation comprend en particulier
le pétrissage d'un mélange contenant
- le composé organique hydrophobe à encapsuler,
- une maltodextrine et/ou un sirop de glucose déshydraté obtenus, par hydrolyse acide ou enzymatique, à partir d'un amidon de pois présentant une teneur en amylose comprise entre 25 % et 50%, exprimé en poids sec par rapport au poids sec d'amidon, et
- une quantité d'eau insuffisante pour dissoudre complètement ladite maltodextrine et/ou ledit sirop de glucose déshydraté, et
l'élimination de l'eau par séchage.

Le procédé décrit ci-dessus peut être mis en oeuvre par exemple dans une extrudeuse.

L'étape de séchage peut s'effectuer par exemple dans une étuve ou dans un granulateur.

L'utilisation de l'agent d'encapsulation selon l'invention permet d'obtenir des rendements d'encapsulation supérieurs à 70%, voire supérieurs à 80% et même supérieurs à 90%.

Les exemples ci-après montrent que les maltodextrines et sirops de glucose déshydratés de pois permettent d'encapsuler des composés hydrophobes tels que des arômes et/ou des principes actifs avec un haut rendement, et de protéger les substances encapsulées efficacement contre certains facteurs environnants. L'efficacité de cette protection est équivalente ou supérieure à celle observée pour les maltodextrines et sirops de glucose issus d'autres amidons.

### Exempte 1 :

On a préparé des compositions comprenant en tant qu'agent d'encapsulation des maltodextrines ou des sirops de glucose déshydratés de pois (selon l'invention) ou de maïs (à titre comparatif), un émulsifiant et, en tant que molécule à encapsuler, le limonène.

Les maltodextrines et sirops de glucose déshydratés de maïs et de pois ont été obtenus par hydrolyse enzymatique de l'amidon, suivant des procédures connues de l'homme du métier.

Le DE des agents d'encapsulation a été déterminé grâce au test de réduction des sucres décrit dans la littérature (F*ood Chemicals Codex,* 4ème édition, 1^{er} juillet 1996. Section 5, *General Tests and Assays, Appendix X: Carbohydrates (Starches, Sugars, and Related Substances*).

Les maltodextrines (MD) et sirops de glucose déshydratés (SDG) utilisées sont les suivantes :
- Maltodextrine de maïs de DE 17,4 : Glucidex^{®} 17
- Maltodextrine de maïs de DE 12 : Glucidex^{®} 12
- Maltodextrine de pois de DE 7
- Maltodextrine de pois de DE 13,5
- Maltodextrine de pois de DE 17
- Sirop de glucose déshydraté de pois de DE 21,5

L'émulsifiant utilisé est un amidon fluidifié, plus particulièrement un amidon de maïs « waxy » modifié par greffage de groupements octényl succinate. Cet émulsifiant est commercialisé par la Demanderesse sous la dénomination CLEARGUM^{®} CO01.

**Tableau 1 : composition d'encapsulation**

| | Quantité en % | Quantité en masse (g) pour 40% de matière sèche |
|---|---|---|
| MD | 21 | 105g |
| Emulsifiant | 15 | 75g |
| Limonène | 4 | 20g |
| Eau déminéralisée | 60 | 300g |

Le protocole d'encapsulation est le suivant : Une pré-émulsion est obtenue par dissolution de la maltodextrine et/ou du sirop de glucose et de l'émulsifiant sous agitation lente dans de l'eau déminéralisée à 70°C pendant 10 min. On prépare parallèlement une solution contenant la molécule à encapsuler, on la préchauffe à 30°C, puis on l'ajoute à la pré-émulsion et on maintient une agitation vive pendant 10 min.

La pré-émulsion est soumise pendant 10 min et à une température de 5°C (immersion dan un bain marie) à un cisaillement important à une vitesse de 24 000 tpm dans un appareil POLYTRON PT 45/2M.

L'atomisation (atomiseur Lab Plant SD05) est effectuée sur l'émulsion préchauffée à 60°C avec une température d'entrée à 175°C et par l'utilisation d'une buse d'injection de 1 mm de diamètre autorisant une pression de l'ordre de 2 bars en son sein.

Afin de mesurer le taux d'encapsulation, une mesure de la rétention du limonène est effectuée. L'étude est effectuée de façon standard telle que décrit dans la littérature (Inglett G. E., Gelbman P., and Gary A. Reineccius (1988) "Encapsulation of Orange Oil: Use of Oligosaccharides from α-Amylase Modified Starches of Maize, Rice, Cassava, and Potato." In "Flavor Encapsulation," eds. Sara J. Risch and Gary A. reineccius, chap. 4, pp. 29-36.). Le taux de rétention reflète la quantité de limonène qui a été encapsulée au cours de l'atomisation.

La fraction de limonène non encapsulée correspond au limonène en surface des particules obtenues après atomisation et au limonène évaporé au cours de l'atomisation.

Après atomisation, la poudre obtenue est lavée avec un solvant non-aqueux tel que l'hexane afin de recueillir la portion de limonène en surface des particules.

La poudre atomisée est ensuite solubilisée afin de recueillir cette fois le limonène encapsulé.

Pour chaque portion, le limonène et l'oxyde de limonène sont mis en solution dans l'acétone puis purifiés et dosés par chromatographie en phase gazeuse (chromatographe VARIAN 8200 CX autosampler) à partir d'une colonne DB1 (Lg 30 m, diam. Int. 0,32 mm, épaisseur du film 1 µm). La chromatographie s'effectue sous un gradient de température de 60 à 250°C à raison de 7°C/min. Le gaz vecteur est l'hélium à une pression de 10 psi (68947.5728 Pa). La calibration s'effectue avec une solution mère d'étalon interne d'acétone à 0,15 g/l de 4-méthyle-2-pentanone (MIBC). Les solutions mères sont des solutions d'acétone à 2g/l de limonène et à lg/l d'oxyde de limonène.

La quantité de limonène évaporé au cours de l'atomisation est déduite à partir de la quantité de limonène total, du limonène en surface des particules et du limonène encapsulé.

Le taux de rétention du limonène correspond au rapport du limonène encapsulé sur le limonène non encapsulé.

**Tableau 2 : Taux de rétention du limonène**

| Support | | Limonène (%) |
|---|---|---|
| MD maïs | DE12 | 67 |
| | DE 17 | 67 |
| | DE 7 | 74 |
| MD pois | DE 13 | 63 |
| | DE 17 | 63 |
| SGD pois | DE 21,5 | 74 |

Les taux de rétention de limonène observés pour les maltodextrines de pois sont comparables à ceux obtenus avec les maltodextrines de maïs (voir tableau 2). Ainsi, les maltodextrines de pois permettent un enrobage du substrat à un taux comparable à celui observé pour les maltodextrines de maïs. Ce taux d'encapsulation est même meilleur pour les maltodextrines de pois de DE 7. En effet, dans ce dernier cas, le taux de rétention du limonène est significativement supérieur au taux de rétention observé pour les maltodextrines de maïs. On remarque par ailleurs, que le taux de rétention est aussi important dans le cas de sirop de glucose déshydraté de pois DE 21,5.

### Exemple 2 :

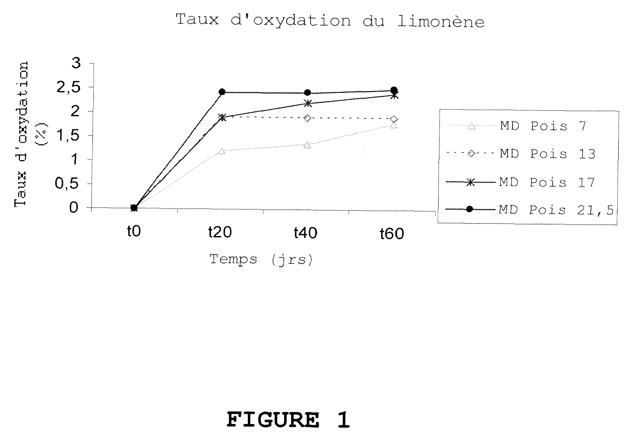

Une composition identique à l'exemple 1 est utilisée pour une encapsulation de limonène par atomisation. Afin de déterminer la qualité de l'encapsulation du limonène ou le pouvoir encapsulant des maltodextrines (MD) et/ou des sirops de glucose déshydraté (SGD) de pois ou de maïs, c'est-à-dire le pouvoir de protection contre les agressions de l'environnement extérieur, une mesure de l'oxydation du limonène en fonction du temps est effectuée (voir figure 1 et tableau 3). Le limonène encapsulé est incubé à une température de 70°C en étuve pendant 20, 40 et 60 jours. Le limonène et l'oxyde de limonène sont mis en solution dans l'acétone puis purifiés et dosés par chromatographie en phase gazeuse (chromatographe VARIAN 8200 CX autosampler) selon les conditions énoncées dans l'exemple 1.

**Tableau 3:**

| **Support** | **%oxyde de limonène/ 100% limonène** | | | |
|---|---|---|---|---|
| | **t0** | **t20** | **t40** | **t60** |
| MD Maïs 12 | 0 | 1,79 | 1,79 | 1,80 |
| MD Maïs 17 | 0 | 1,79 | 1,94 | 1,98 |
| MD Pois 7 | 0 | 1,22 | 1,35 | 1,78 |
| MD Pois 13 | 0 | 1,90 | 1,90 | 1,90 |
| MD Pois 17 | 0 | 1,90 | 2,22 | 2,41 |
| SDG Pois 21,5 | 0 | 2,43 | 2,43 | 2, 50 |

La mesure du taux d'oxydation du limonène reflète une qualité d'encapsulation comparable entre les maltodextrines et/ou des sirops de glucose de maïs et de pois vis-à-vis de l'oxydation du limonène c'est-à-dire un pouvoir encapsulant comparable.

Afin de déterminer le type d'encapsulation observé lors de l'utilisation de maltodextrines et/ou de sirops de glucose de pois comme support d'encapsulation, une comparaison de l'évolution de l'oxydation du limonène en fonction du DE des maltodextrines et/ou des sirops de glucose de pois est effectuée (figure 1).

Il est classiquement décrit dans la littérature que lors de l'utilisation de maltodextrines et de sirops de glucose de maïs ou de pommes de terre comme support d'encapsulation, une augmentation d'oxydation du limonène est observée lorsque les DE des maltodextrines et des sirops de glucose diminuent (Reineccius G. A. (1988) « Spray drying of Food Flavors" In "Flavor Encapsulation," eds. Sara J. Risch and Gary A. reineccius, chap. 7, pp. 55-66.). Ainsi, la qualité de l'encapsulation par des maltodextrines et des sirops de glucose de maïs ou de pommes de terre augmente lorsque leur DE augmente.

Dans le cas des maltodextrines et des sirops de glucose de pois, un phénomène inverse est observé (voir figure 1). En effet, lors d'une encapsulation par atomisation avec des maltodextrines ou de sirops de glucose de pois comme agent d'encapsulation, l'oxydation du limonène diminue lorsque les DE des maltodextrines et/ou de sirops de glucose diminuent. Autrement dit, le pouvoir encapsulant augmente lorsque les DE des maltodextrines et/ou de sirops de glucose de pois diminuent. De plus, les maltodextrines de pois permettent une protection du substrat encapsulé face à l'environnement extérieur meilleure que celles obtenues avec les sirops de glucose déshydratés de pois.

Ainsi, les phénomènes observés au cours d'une encapsulation par des maltodextrines et/ou des sirops de glucose de maïs (ou de pommes de terre) et ceux observés au cours d'une encapsulation par des maltodextrines et/ou des sirops de glucose de pois n'obéissent pas aux mêmes principes

### Exemple_3

Une composition d'encapsulation est réalisée afin d'effectuer l'encapsulation du menthol par des maltodextrines de pois (selon l'invention) ou de maïs (à titre comparatif) ou par une β-cyclodextrine (à titre comparatif) par une technique d'encapsulation utilisée uniquement pour les cyclodextrines. L'encapsulation est effectuée par pétrissage, c'est-à-dire en malaxant dans un pétrin à double enveloppe une composition comportant 68% d'agent encapsulant (maltodextrine ou cyclodextrine), 22,7% d'eau et 9,3% de menthol.

L'arôme est ajouté à l'agent encapsulant progressivement. Le mélange est malaxé durant 5 à 10 min puis subit une phase de séchage à 70°C. La composition séchée comporte 88% de maltodextrines de pois ou de maïs ou de β-cyclodextrines et 12% de menthol.

Une analyse calorimétrique différentielle (ACD ou DSC ; appareil METTLER DSC 30) est effectuée afin de mesurer le taux de complexation entre les maltodextrines ou les β-cyclodextrines et le menthol.

Au cours de cette étude, le mélange est soumis à une phase de réchauffement au cours de laquelle il subit un changement d'état. La température de transition vitreuse (Tg) est ainsi déterminée de même que l'amplitude du signal (ΔCp) qui est directement proportionnel au taux de complexation entre l'agent encapsulant et le menthol.

Le protocole suivi au cours de cette étude est le suivant. L'échantillon est maintenu à -20°C pendant 2 min puis il subit un changement de température de -20 à 180°C à une vitesse de 10°C/min.

Le taux d'encapsulation obtenu pour les β-cyclodextrines est de 100% (résultat non montré). Ce pourcentage est classiquement obtenu pour une encapsulation par pétrissage du menthol par les β-cyclodextrines.

**Tableau 4**

| **Support** | **DSC** | | **Taux de complexes (%)** |
|---|---|---|---|
| | **Tg (°C)** | **ΔCp (j/g/°c)** | |
| MD de pois DE7 | 83,4 | 0,035 | 100 |
| | 103,7 | 0,1 | |
| MD de pois DE 13,5 | 96,65 | 0,065 | 93,90 |
| MD de pois DE17 | 82,5 | 0,05 | 100 |
| | 105,3 | 0,12 | |
| MD de maïs DE 12 | 105, 4 | 0,1 | 55,20 |
| MD de maïs DE 17 | 107,4 | 0,11 | 64,40 |

Lorsque le taux de complexation mesuré est de 100%, une nouvelle analyse est effectuée afin de confirmer la mesure.

Cette analyse montre un taux de complexation maltodextrine de pois/menthol de l'ordre de 94 à 100% alors que le taux observé pour les complexes maltodextrine de maïs/menthol est beaucoup plus faible de 55 à 64%.

## Revendications

1. Utilisation d'une maltodextrine ou d'un sirop de glucose obtenus, par hydrolyse acide ou enzymatique, à partir d'un amidon de pois présentant une teneur en amylose comprise entre 25% et 50%, exprimé en poids sec par rapport au poids sec d'amidon, pour l'encapsulation de composés organiques hydrophobes.

2. Utilisation selon la revendication 1, **caractérisée par le fait que** l'amidon de pois présente une teneur en amylose comprise entre 30% et 40%, de préférence comprise entre 35 et 40%, et plus préférentiellement entre 35 et 38%, exprimé en poids sec par rapport au poids sec d'amidon.

3. Utilisation selon la revendication 1 ou 2, **caractérisé en ce que** le sirop de glucose présente un dextrose équivalent (DE) inférieur à 30.

4. Utilisation selon la revendication 1 ou 2, **caractérisé en ce que** la maltodextrine présente un dextrose équivalent (DE) inférieur à 18 et de préférence compris entre 3 et 7.

5. Utilisation selon l'une quelconque des revendications précédentes, **caractérisé en ce que** les composés à encapsuler sont choisis parmi les arômes, les colorants, les vitamines liposolubles, les molécules odorantes, les édulcorants intenses, les matières grasses.

6. Utilisation selon la revendication 5, **caractérisée en ce que** les composés à encapsuler sont choisis parmi les acides gras, les monoglycérides, le décanal, l'octanal, l'hexanal, le butanol, la menthone, la fenchone, le limonène, le naphtol, les oléorésines d'épice, les huiles essentielles des saveurs alliacées, l'aldéhyde benzoïque, le diacétyle (2,3-butanedione), la vanilline, le thymol, le menthol, le camphre, le géraniol, la carvone, la δ-heptalactone, la δ-nonalactone, la δ-decalactone, la δ-dodecalactone, la γ-decalactone, la γ-dodecalactone et la quinoléine.

7. Procédé d'encapsulation de composés organiques hydrophobes, comprenant
- la dissolution d'une maltodextrine et/ou d'un sirop de glucose obtenus, par hydrolyse acide ou enzymatique, à partir d'un amidon de pois présentant une teneur en amylose comprise entre 25% et 50%, exprimé en poids sec par rapport au poids sec d'amidon, dans un solvant aqueux contenant un agent émulsionnant,
- l'addition d'une solution du composé organique hydrophobe à encapsuler dans un solvant organique,
- l'émulsification du mélange obtenu par application de forces de cisaillement, et
- le séchage de l'émulsion obtenue, de préférence par atomisation.

8. Procédé d'encapsulation de composés organiques hydrophobes, comprenant le pétrissage d'un mélange contenant
- le composé organique hydrophobe à encapsuler,
- une maltodextrine et/ou un sirop de glucose déshydraté obtenus, par hydrolyse acide ou enzymatique, à partir d'un amidon de pois présentant une teneur en amylose comprise entre 25 % et 50%, exprimé en poids sec par rapport au poids sec d'amidon, et
- une quantité d'eau insuffisante pour dissoudre complètement ladite maltodextrine et/ou ledit sirop de glucose déshydraté,
et l'élimination de l'eau par séchage.

9. Procédé selon la revendication 7 ou 8, **caractérisé par le fait que** l'amidon de pois présente une teneur en amylose comprise entre 30 % et 40%, de préférence comprise entre 35 et 40%, et plus préférentiellement entre 35 et 38%, exprimé en poids sec par rapport au poids sec d'amidon.

10. Procédé selon la revendication 7 ou 8, **caractérisé par le fait que** le sirop de glucose présente un dextrose équivalent (DE) inférieur à 30.

11. Procédé selon la revendication 7 ou 8, **caractérisé par le fait que** la maltodextrine présente un dextrose équivalent (DE) inférieur à 18, de préférence compris entre 3 et 7.

12. Procédé selon la revendication 7 ou 8, **caractérisé par le fait que** les composés à encapsuler sont choisis parmi les acides gras, les monoglycérides, le décanal, l'octanal, l'hexanal, le butanol, la menthone, la fenchone, le limonène, le naphtol, les oléorésines d'épice, les huiles essentielles des saveurs alliacées, l'aldéhyde benzoïque, le diacétyle (2,3-butanedione), la vanilline, le thymol, le menthol, le camphre, le géraniol, la carvone, la δ-heptalactone, la δ-nonalactone, la δ-decalactone, la δ-dodecalactone, la γ-decalactone, la γ-dodecalactone et la quinoléine.

## Patentansprüche

1. Verwendung eines Maltodextrins oder eines Glucosesirups, erhalten durch saure oder enzymatische Hydrolyse, ausgehend von einer Erbsenstärke mit einem Amylosegehalt, umfassend zwischen 25 % und 50 %, ausgedrückt als Trockengewicht, bezogen auf das Trockengewicht der Stärke, zur Einkapselung hydrophober organischer Verbindungen.

2. Verwendung nach Anspruch 1, **dadurch gekennzeichnet, dass** die Erbsenstärke einen Amylosegehalt aufweist, umfassend zwischen 30 % und 40 %, vorzugsweise zwischen 35 und 40 % und mehr bevorzugt zwischen 35 und 38 %, ausgedrückt als Trockengewicht, bezogen auf das Trockengewicht der Stärke.

3. Verwendung nach Anspruch 1 oder 2, **dadurch gekennzeichnet, dass** der Glucosesirup ein Dextroseäquivalent (DE) unter 30 aufweist.

4. Verwendung nach Anspruch 1 oder 2, **dadurch gekennzeichnet, dass** das Maltodextrin ein Dextroseäquivalent (DE) unter 18 und vorzugsweise ein Glucoseäquivalent, umfassend zwischen 3 und 7, aufweist.

5. Verwendung nach einem der vorhergehenden Ansprüche, **dadurch gekennzeichnet, dass** die Einkapselungsverbindungen ausgewählt sind aus Aromen, Farbstoffen, fettlöslichen Vitaminen, Duftmolekülen, Intensivsüßmitteln, Fetten.

6. Verwendung nach Anspruch 5, **dadurch gekennzeichnet, dass** die Einkapselungsverbindungen ausgewählt sind aus Fettsäuren, Monoglyceriden, Decanal, Octanal, Hexanal, Butanol, Menthon, Fenchon, Limonen, Naphthol, Gewürzoleoresinen, ätherischen Ölen mit Zwiebelgewächsgeschmack, Benzaldehyd, Diacetyl (2,3-Butandion), Vanillin, Thymol, Menthol, Kampfer, Geraniol, Carvon, δ-Heptalacton, δ-Nonalacton, δ-Decalacton, δ-Dodecalacton, γ-Decalacton, γ-Dodecalacton und Chinolin.

7. Einkapselungsverfahren für hydrophobe organische Verbindungen, umfassend:
- Lösen eines Maltodextrins und/oder eines Glucosesirups, erhalten durch saure oder enzymatische Hydrolyse, ausgehend von einer Erbsenstärke mit einem Amylosegehalt, umfassend zwischen 25 % und 50 %, ausgedrückt als Trockengewicht, bezogen auf das Trockengewicht der Stärke, in einem wässrigen Lösungsmittel, das ein Emulgiermittel enthält,
- Zugabe einer Lösung der hydrophoben organischen Verbindung, die einzukapseln ist, in einem organischen Lösungsmittel,
- Emulgieren des erhaltenen Gemischs durch Anwendung von Scherkraft, und
- Trocknen der erhaltenen Emulsion, vorzugsweise durch Zerstäuben.

8. Einkapselungsverfahren für hydrophobe organische Verbindungen, umfassend das Kneten eines Gemischs, umfassend:
- die hydrophobe organische Verbindung, die einzukapseln ist,
- ein Maltodextrin und/oder ein dehydratisierten Glucosesirup, erhalten durch saure oder enzymatische Hydrolyse, ausgehend von einer Erbsenstärke mit einem Amylosegehalt, umfassend zwischen 25 % und 50 %, ausgedrückt als Trockengewicht, bezogen auf das Trockengewicht der Stärke, und
- eine Wassermenge, die nicht ausreichend ist, um das Maltodextrin und/oder den dehydratisierten Glucosesirup aufzulösen,
und Entfernen des Wassers durch Trocknen.

9. Verfahren nach Anspruch 7 oder 8, **dadurch gekennzeichnet, dass** die Erbsenstärke einen Amylosegehalt aufweist, umfassend zwischen 30 % und 40 %, vorzugsweise zwischen 35 und 40 % und mehr bevorzugt zwischen 35 und 38 %, ausgedrückt als Trockengewicht bezogen auf das Trockengewicht der Stärke.

10. Verfahren nach Anspruch 7 oder 8, **dadurch gekennzeichnet, dass** der Glucosesirup ein Dextroseäquivalent (DE) unter 30 aufweist.

11. Verfahren nach Anspruch 7 oder 8, **dadurch gekennzeichnet, dass** das Maltodextrin ein Dextroseäquivalent (DE) unter 18 und vorzugsweise ein Glucoseäquivalent, umfassend zwischen 3 und 7, aufweist.

12. Verfahren nach Anspruch 7 oder 8, **dadurch gekennzeichnet, dass** die Einkapselungsverbindungen ausgewählt sind aus Fettsäuren, Monoglyceriden, Decanal, Octanal, Hexanal, Butanol, Menthon, Fenchon, Limonen, Naphthol, Gewürzoleoresinen, ätherischen Ölen mit Zwiebelgewächsgeschmack, Benzaldehyd, Diacetyl (2,3-Butandion), Vanillin, Thymol, Menthol, Kampfer, Geraniol, Carvon, δ-Heptalacton, δ-Nonalacton, δ-Decalacton, δ-Dodecalacton, γ-Decalacton, γ-Dodecalacton und Chinolin.

## Claims

1. Use of a maltodextrin or a glucose syrup obtained, by acid or enzymatic hydrolysis, from a pea starch having an amylose content between 25% and 50%, expressed as dry weight relative to the dry weight of starch, for the encapsulation of hydrophobic organic compounds.

2. Use according to claim 1 **characterised in that** the pea starch has an amylose content between 30% and 40%, preferably between 35 and 40%, and more preferentially between 35 and 38%, expressed as dry weight relative to the dry weight of starch.

3. Use according to claim 1 or 2, **characterised in that** the maltodextrine has a dextrose equivalent (DE) less than 30.

4. Use according to claim 1 or 2, **characterised in that** the glucose syrup has a dextrose equivalent (DE) less than 18 and preferably between 3 and 7.

5. Use according to any of the above claims, **characterised in that** the compounds to be encapsulated are chosen from flavourings, colorants, fat-soluble vitamins, odorous molecules, intense sweeteners, fats.

6. Use according to claim 5, **characterised in that** the compounds to be encapsulated are chosen from fatty acids, monoglycerides, decanal, octanal, hexanal, butanol, menthone, fenchone, limonene, naphthol, spice oleoresins, alliaceous flavour essential oils, benzoic aldehyde, diacetyl (2,3-butanedione), vanillin, thymol, menthol, camphor, geraniol, carvone, δ-heptalactone, δ-nonalactone, δ-decalactone, δ-dodecalactone, γ-decalactone, γ-dodecalactone and quinoline.

7. Method for encapsulating hydrophobic organic compounds, comprising
- dissolution of a maltodextrin and/or a glucose syrup obtained, by acid or enzymatic hydrolysis, from a pea starch having an amylose content between 25% and 50%, expressed as dry weight relative to the dry weight of starch, in an aqueous solvent containing an emulsifying agent,
- addition of a solution of the hydrophobic organic compound to be encapsulated in an organic solvent,
- emulsification of the mixture obtained by applying shear forces, and
- drying of the emulsion obtained, preferably by spray-drying.

8. Method for encapsulating hydrophobic organic compounds, comprising mixing of a mixture containing
- the hydrophobic organic compound to be encapsulated,
- a maltodextrin and/or a glucose syrup obtained, by acid or enzymatic hydrolysis, from a pea starch having an amylose content between 25% and 50%, expressed as dry weight relative to the dry weight of starch, and
- an insufficient quantity of water to completely dissolve said maltodextrin and/or said dehydrated glucose syrup,
and removal of water by drying.

9. Method according to claim 7 or 8, **characterised in that** the pea starch has an amylose content between 30% and 40%, preferably between 35 and 40%, and more preferentially between 35 and 38%, expressed as dry weight relative to the dry weight of starch.

10. Method according to claim 7 or 8, **characterised in that** the maltodextrine has a dextrose equivalent (DE) less than 30.

11. Method according to claim 7 or 8, **characterised in that** the glucose syrup has a dextrose equivalent (DE) less than 18 and preferably between 3 and 7.

12. Method according to claim 7 or 8, **characterised in that** the compounds to be encapsulated are chosen from fatty acids, monoglycerides, decanal, octanal, hexanal, butanol, menthone, fenchone, limonene, naphthol, spice oleoresins, alliaceous flavour essential oils, benzoic aldehyde, diacetyl (2,3-butanedione), vanillin, thymol, menthol, camphor, geraniol, carvone, δ-heptalactone, δ-nonalactone, δ-decalactone, δ-dodecalactone, γ-decalactone, γ-dodecalactone and quinoline.
